# EUROPEAN PATENT SPECIFICATION

(11) **EP 4 306 163 B1**
(45) Date of publication and mention of the grant of the patent: **25.12.2024**
(21) Application number: 23184720.3
(22) Date of filing: 11.07.2023
(51) Int. Cl.: A61N 5/10

(54) **BRACHYTHERAPY APPLICATOR**
BRACHYTHERAPIEAPPLIKATOR
APPLICATEUR DE BRACHYTHÉRAPIE

(30) Priority: 13.07.2022 US 202217863789
(43) Date of publication of application: 17.01.2024
(73) Proprietor: VARIAN MEDICAL SYSTEMS HAAN GMBH, 42781 Haan (DE)
(72) Inventor: JÄNSCH, Judith, 42109 Wuppertal (DE); OTT, Michael, 92676 Speinshart (DE); DESELAERS, Ruth, 40822 Mettmann (DE); KNÖFEL, Thomas, 92690 Pressath (DE); RUPPRECHT, Josef, 92681 Erbendorf (DE); GERBER, Christian, 42781 Haan (DE)
(74) Representative: Goddard, Frances Anna

(56) References cited:
- US-A1- 2007 270 627
- US-A1- 2011 257 459
- US-A1- 2018 085 597
- US-B1- 10 738 924

## Description

### Field

The present disclosure relates to an applicator for insertion into a body cavity for brachytherapy treatment. In particular, although not exclusively, the present disclosure relates to a brachytherapy applicator and a method of manufacturing an applicator, in particular a brachytherapy applicator.

The applicator taught by US 2018/085597 is devoid of grooves and projections as set out in the present claims.

### SUMMARY

The present invention provides a method of manufacturing an applicator as claimed in the claims hereto. Suitably, a method of manufacturing an applicator according to the present invention is a method of manufacturing a brachytherapy applicator. Furthermore, the present invention provides a brachytherapy applicator as claimed in the claims hereto.

At least one example embodiment of the present invention relates to a method of manufacturing a brachytherapy applicator.

In at least one example embodiment, the method includes the steps defined in claim 1.

In at least one example embodiment, the providing the shaft includes forming the shaft by additive manufacturing. In at least one example embodiment, the providing a cap includes forming the cap by additive manufacturing. In at least one example embodiment, the providing the base portion includes forming the base portion by additive manufacturing.

In at least one example embodiment, the forming an applicator further includes applying a resin between the shaft and the cap, and curing the resin and/or, the forming an applicator further includes applying the resin between the shaft and the base portion, and curing the resin. For example, the resin may be applied to the top surface of the shaft and/or to the mating surface of the cap. The resin may also be introduced into the first groove, the second groove, and/or the one or more holes of the shaft. After applying the resin and connecting the cap to the shaft, the resin is cured such that the cap is secured to the shaft.

The first end of the shaft comprises a top surface. Suitably, the top surface defines a first groove and a second groove and the second groove circumscribes the first groove.

The cap includes a mating surface configured to be coupled to the top surface of the shaft. Suitably, a first plurality of projections extend from the mating surface, and a second plurality of projections extend from the mating surface and circumscribe the first plurality of projections.

The step of connecting the cap to the first end of the shaft includes engaging the first plurality of projections of the cap with the first groove of the shaft and engaging the second plurality of projections of the cap with the second groove of the shaft.

In at least one example embodiment, the first end of the shaft comprises a top surface and the top surface defines one or more holes. Suitably, the cap includes a mating surface configured to be coupled to the top surface of the shaft and one or more positioning elements extending from the mating surface.

In at least one example embodiment, the connecting the cap to the first end of the shaft includes inserting the one or more positioning elements of the cap into the one or more holes of the shaft.

In at least one example embodiment, the method further comprises forming an inlet in a side of the shaft adjacent the first end of the shaft, forming an outlet in another side of the shaft, and applying a resin between the shaft and the cap through the inlet.

In at least one example embodiment, the forming the applicator further comprises coupling a plurality of connectors to the base portion and applying a resin between the connectors and the base portion.

In at least one example embodiment, the applicator includes a primary channel and one or more secondary channels. The primary channel may extend through a center of the applicator through the base portion, the shaft, and the cap. The one or more secondary channels may circumscribe the primary channel. For example, the one or more secondary channel may be equidistantly spaced apart about the primary channel. In at least one example embodiment, the one or more secondary channel extends from a second section of the base portion, along the shaft, and at least partially through the cap. The one or more secondary channels may comprise two to eight channels. In at least one example embodiment, the one or more secondary channels comprises six channels.

In at least one example embodiment, the shaft includes a first central channel extending from the first end to the second end. The shaft may comprise a first plurality of lumens extending from the first end to the second end. In at least one example embodiment, the first plurality of lumens may be positioned between the first central channel and the exterior surface of the shaft. For example, the first plurality of lumens may be positioned between the first groove and the second groove. The first plurality of lumens may form at least a portion of the one or more secondary channels extending through the shaft. The first plurality of lumens are suitably positioned circumferentially about the first central channel. The first plurality of lumens may be equidistantly spaced about the first central channel. The first plurality of lumens may comprise two to eight lumens. In at least one example embodiment, the first plurality of lumens comprises six lumens.

The cap suitably includes a second central channel extending through a center of the cap. Suitably, the second central channel is configured to be coupled to the first central channel of the shaft. For example, the second central channel may form at least a portion of the primary channel extending through the cap. Suitably, the cap further comprises a plurality of holes extending at least partially through the cap. Suitably, the plurality of holes are positioned circumferentially about the second central channel and configured to be coupled to the first plurality of lumens. In at least one example embodiment, the plurality of holes of the cap may comprise two to eight holes. In at least one example embodiment, the plurality of holes may comprise six holes.

The base portion may include a third central channel extending through a center of the base portion and a second plurality of lumens extending through the base portion. In at least one example embodiment, the second plurality of lumens comprises two to eight lumens. In at least one example embodiment, the second plurality of lumens comprises six lumens. The third central channel is suitably configured to be coupled to the first central channel and the second plurality of lumens are suitably positioned circumferentially about the third central channel and configured to be coupled to the first plurality of lumens. In an embodiment of the invention, the shaft includes a first plurality of projections and a second plurality of projections extending from the bottom surface thereof. The first plurality of projections may circumscribe the first central channel. The second plurality of projections may circumscribe the first plurality of projections. In at least one example embodiment, the second plurality of projections may be positioned adjacent an outer perimeter of the shaft. For example, the second plurality of projections may be between the outer perimeter of the shaft and the first plurality of lumens. In at least one example embodiment, the first plurality of projections is configured to engage the first groove of the base portion and the second plurality of projections is configured to engage the second groove of the base portion such that the base portion is secured to the second end of the shaft. In at least one example embodiment, the shaft includes one or more positioning elements extending from the bottom surface. The one or more positioning elements may be positioned between the first plurality of projections and the second plurality of projections. In at least one example embodiment, the one or more holes in the mating surface of the base portion are configured to receive the one or more positioning elements. In at least one example embodiment, the one or more positioning elements may comprise three positioning elements. In at least one example embodiment, the one or more positioning elements are spaced equidistantly about the first central channel. In at least one example embodiment, the one or more positioning elements may comprise a flexible material.

In at least one example embodiment, the shaft, the cap, and/or the base portion comprise a resin or a plastic material. In at least one example embodiment, the shaft, the cap, and/or the base portion of the applicator may comprise transparent, non-transparent, or a combination of both transparent and non-transparent materials. In at least one example embodiment, surfaces of or materials forming the applicator may also be provided with antibacterial additives such as copper, silver, and/or graphene.

In at least one example embodiment, the method further comprises cleaning the shaft, the base portion, and/or the cap before the forming the applicator.

At least one example embodiment relates to a brachytherapy applicator for insertion into a body cavity.

In at least one example embodiment, the brachytherapy applicator includes a shaft and a cap. The shaft has a first end and a second end opposite the first end. The shaft also includes a top surface adjacent the first end of the shaft. The top surface defines a first groove and a second groove. In at least one example embodiment, the first groove circumscribes a first central channel extending through the shaft from the first end to the second end. The first central channel may form at least a portion of the primary channel. The second groove may circumscribe the first groove. For example, the second groove may be positioned adjacent an exterior surface of the shaft. In at least one example embodiment, the top surface of the shaft defines one or more holes. The one or more holes may be positioned between the first groove and the second groove. In at least one example embodiment, the one or more holes comprises three holes. The one or more holes may be spaced equidistantly about a circumference of the top surface of the shaft.

The cap is suitably configured to be coupled to the first end of the shaft. The cap includes a mating surface configured to be coupled to the top surface of the shaft, a first plurality of projections extending from the mating surface and a second plurality of projection extending from the mating surface. The first plurality of projections are configured to engage the first groove. The second plurality of projections suitably circumscribe the first plurality of projections and are configured to engage the second groove. In at least one example embodiment, the second plurality of projections may be positioned adjacent an outer perimeter of the first side of the cap.

In at least one example embodiment, the cap comprises one or more positioning elements extending from the mating surface. The one or more positioning elements may be positioned between the first plurality of projections and the second plurality of projections. Suitably, the top surface of the shaft defines one or more holes configured to receive the one or more positioning elements. In at least one example embodiment, the one or more positioning elements may comprise three positioning elements. The one or more positioning elements may be equidistantly spaced about a circumference of the cap. In at least one example embodiment, the one or more positioning elements may comprise a flexible material. The flexible material may enable the one or more positioning elements to be inserted into the one or more holes of the shaft by a snap-fit and/or a form-fit.

In at least one example embodiment, the shaft includes a first central channel extending from the first end to the second end and a first plurality of lumens extending from the first end to the second end. The first plurality of lumens are suitably positioned circumferentially about the first central channel. Suitably, the cap includes a second central channel extending through a center of the cap and a plurality of holes extending at least partially through the cap. The second central channel is suitably configured to be coupled to the first central channel. Suitably, the plurality of holes are positioned circumferentially about the second central channel and configured to be coupled to the first plurality of lumens.

In at least one example embodiment, the second end of the shaft includes a base portion. The base portion may include a first section, a second section, and a third section. The second section may be positioned between the first section and the third section of the base portion. In some example embodiments, the base portion and the shaft are a single unitary piece. In other example embodiments, the base portion may be a separate piece configured to be coupled to the second end of the shaft. In such embodiments, the first section of the base portion is configured to be coupled to the second end of the shaft.

In at least one example embodiment, the base portion suitably includes a first section configured to be coupled to the second end of the shaft; a second section including an exterior surface; a third section; a third central channel extending through the first section, the second section, and the third section; and a second plurality of lumens extending through the first section and the second section of the base portion. Suitably, the exterior surface is at an angle relative to a horizontal axis through the base portion. The second section is suitably between the first section and the third section.

In at least one example embodiment, the angle is greater than or equal to 45°. Suitably, the angle of the exterior surface of the base portion relative to a horizontal axis through the base portion is greater than 90°, suitably greater than 120°, for example, greater than or equal to 135°. Suitably, the angle of the exterior surface of the base portion is at an angle relative to a horizontal axis through the base portion of less than 180°, for example, less than 175°, or less than 165°, or less than 155°. Suitably, the angle of the exterior surface of the base portion is at an angle relative to a horizontal axis through the base portion of around 135°.

In at least one example embodiment, the brachytherapy applicator further comprises a plurality of connectors configured to be at least partially inserted into the first plurality of lumens of the third section of the base portion.

In at least one example embodiment, the brachytherapy applicator further comprises a source of radiation configured to be coupled to the plurality of connectors and to deliver radiation to a target location. The source of radiation may include one or more ports configured to receive one or more wires or cables. For example, a first end of the cable may be coupled to one or the ports and a second end of the cable may be coupled to one of connectors of the base portion.

### BRIEF DESCRIPTION OF THE DRAWINGS

The various features and advantages of the non-limiting embodiments herein may become more apparent upon review of the detailed description in conjunction with the accompanying drawings. The accompanying drawings are merely provided for illustrative purposes and should not be interpreted to limit the scope of the claims. The accompanying drawings are not to be considered as drawn to scale unless explicitly noted. For purposes of clarity, various dimensions of the drawings may have been exaggerated.
FIG. 1A is a front view of a brachytherapy applicator according to at least one example embodiment.
FIG. 1B is a section view of the applicator of FIG. 1A along line I-I according to at least one example embodiment.
FIG. 2 is a top perspective view of a shaft of the applicator of FIG. 1A according to at least one example embodiment.
FIG. 3 is a front perspective view of a cap of the applicator of FIG. 1A according to at least one example embodiment.
FIG. 4 is a sectional view of the cap of FIG. 3 along line IV-IV according to at least one example embodiment.
FIG. 5 is a detailed interior view of the shaft and the cap of the applicator of FIG. 1A according to at least one example embodiment.
FIG. 6 is a detailed interior view of the shaft and the cap of the applicator of FIG. 1A according to at least one example embodiment.
FIG. 7 is a top perspective view of a base portion of the applicator of FIG. 1A according to at least one example embodiment.
FIG. 8 is a detailed section view of a second end of the shaft of the applicator of FIG. 1A according to at least one example embodiment.
FIG. 9A is a detailed section view of the shaft and the base portion of the applicator of FIG. 1A according to at least one example embodiment.
FIG. 9B is a detailed section view of the shaft and the base portion of the applicator of FIG. 1A according to at least one example embodiment.
FIG. 10 is a perspective view of the base portion of FIG. 1A according to at least one example embodiment.
FIG. 11 is a section view of the base portion of FIG. 10 along line XI-XI according to at least one example embodiment.
FIG. 12A is a perspective view of the applicator of FIG. 1A and a source of radiation according to at least one example embodiment.
FIG. 12B is a detailed view of the applicator of FIG. 12 according to at least one example embodiment.
FIG. 13 is a flowchart depicting a method of manufacturing an applicator according to at least one example embodiment.

### DETAILED DESCRIPTION OF EXAMPLE EMBODIMENTS

Some detailed example embodiments are disclosed herein. However, specific structural and functional details disclosed herein are merely representative for purposes of describing some example embodiments. Example embodiments may, however, be embodied in many alternate forms and should not be construed as limited to only example embodiments set forth herein.

Accordingly, while example embodiments are capable of various modifications and alternative forms, example embodiments thereof are shown by way of example in the drawings and will herein be described in detail. It should be understood, however, that there is no intent to limit an example embodiment to the particular forms disclosed, but to the contrary, example embodiments are to cover all modifications and alternatives falling within the scope of an example embodiment. Like numbers refer to like elements throughout the description of the figures.

It should be understood that when an element or layer is referred to as being "on," "connected to," "coupled to," or "covering" another element or layer, it may be directly on, connected to, coupled to, or covering the other element or layer or intervening elements or layers may be present. In contrast, when an element is referred to as being "directly on," "directly connected to," or "directly coupled to" another element or layer, there are no intervening elements or layers present. Like numbers refer to like elements throughout the specification. As used herein, the term "and/or" includes any and all combinations of one or more of the associated listed items.

It should be understood that, although the terms first, second, third, etc. may be used herein to describe various elements, regions, layers and/or sections, these elements, regions, layers, and/or sections should not be limited by these terms. These terms are only used to distinguish one element, region, layer, or section from another region, layer, or section. Thus, a first element, region, layer, or section discussed below could be termed a second element, region, layer, or section without departing from the teachings of example embodiment.

Spatially relative terms (e.g., "beneath," "below," "lower," "above," "upper," and the like) may be used herein for ease of description to describe one element or feature's relationship to another element(s) or feature(s) as illustrated in the figures. It should be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the term "below" may encompass both an orientation of above and below. The device may be otherwise oriented (rotated 90 degrees or at other orientations) and the spatially relative descriptors used herein interpreted accordingly.

The terminology used herein is for the purpose of describing various example embodiment only and is not intended to be limiting of example embodiment. As used herein, the singular forms "a," "an," and "the" are intended to include the plural forms as well, unless the context clearly indicates otherwise. It will be further understood that the terms "includes," "including," "comprises," and/or "comprising," when used in this specification, specify the presence of stated features, integers, steps, operations, and/or elements, but do not preclude the presence or addition of one or more other features, integers, steps, operations, elements and/or groups thereof.

Example embodiments are described herein with reference to cross-sectional illustrations that are schematic illustrations of example embodiment. As such, variations from the shapes of the illustrations are to be expected. Thus, example embodiment should not be construed as limited to the shapes of regions illustrated herein but are to include deviations and variations in shapes.

When the words "about" and "substantially" are used in this specification in connection with a numerical value, it is intended that the associated numerical value include a tolerance of ±10% around the stated numerical value, unless otherwise explicitly defined. Moreover, when the terms "generally" or "substantially" are used in connection with geometric shapes, it is intended that precision of the geometric shape is not required but that latitude for the shape is within the scope of the disclosure. Furthermore, regardless of whether numerical values or shapes are modified as "about," "generally," or "substantially," it will be understood that these values and shapes should be construed as including a manufacturing or operational tolerance (e.g., ±10%) around the stated numerical values or shapes.

Unless otherwise defined, all terms (including technical and scientific terms) used herein have the same meaning as commonly understood by one of ordinary skill in the art to which example embodiment belong. It will be further understood that terms, including those defined in commonly used dictionaries, should be interpreted as having a meaning that is consistent with their meaning in the context of the relevant art and will not be interpreted in an idealized or overly formal sense unless expressly so defined herein.

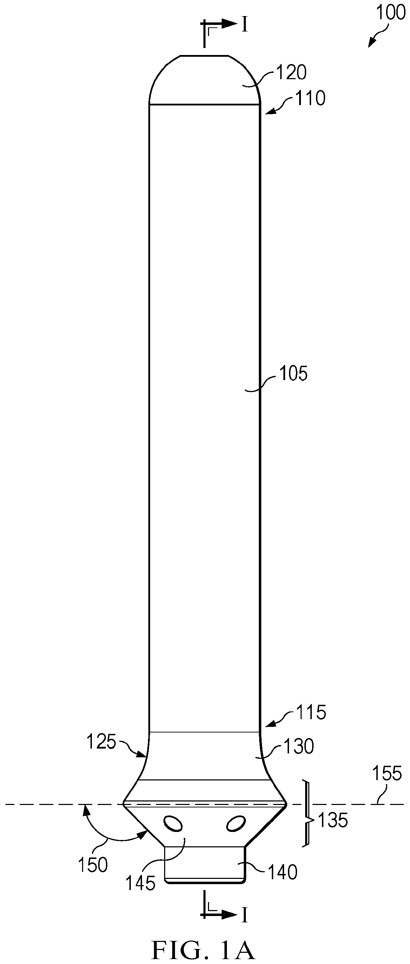

FIG. 1A is a front view of a brachytherapy applicator according to at least one example embodiment.

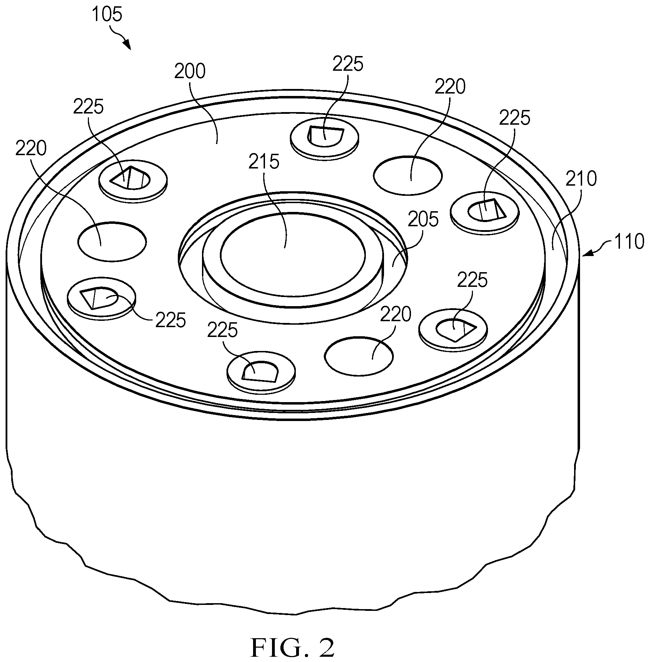

An applicator 100 may be inserted into a body cavity for brachytherapy treatment. The applicator 100 includes a shaft 105 having a first end 110 and a second end 115 opposite the first end 110. In at least one example embodiment, the shaft 105 has a cylindrical shape. In at least one example embodiment, the applicator 100 includes a cap 120 configured to be coupled to the first end 110 of the shaft 105, as will be discussed in relation to FIGS. 2-6, below.

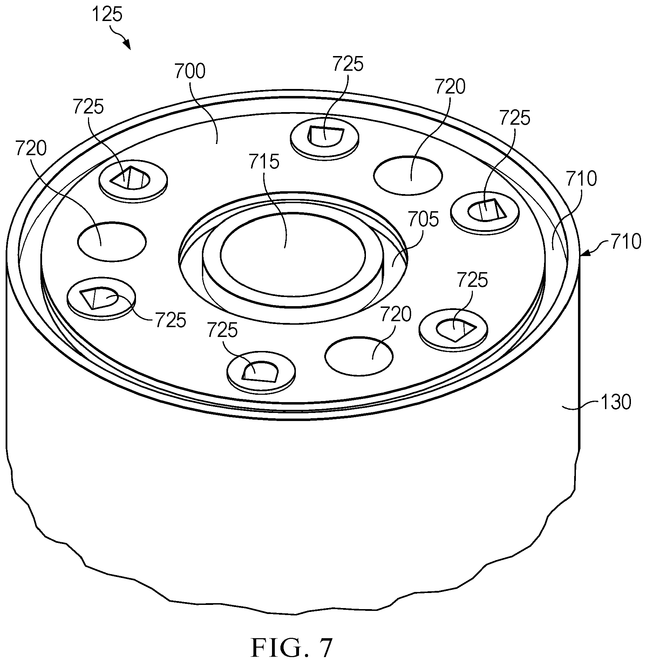

In at least one example embodiment, the applicator 100 includes a base portion 125 at the second end 115 of the shaft 105. In at least one example embodiment, the base portion 125 includes a first section 130, a second section 135, and a third section 140. The second section 135 is positioned between the first section 130 and the third section 140 of the base portion 125. In some example embodiments, the base portion 125 and the shaft 105 are a single unitary piece. In other example embodiments, the base portion 125 may be a separate piece configured to be coupled to the second end 115 of the shaft 105, as will be discussed in relation to FIGS. 7-11, below. In such embodiments, the first section 130 of the base portion 125 is configured to be coupled to the second end 115 of the shaft 105.

In at least one example embodiment, the second section 135 of the base portion 125 includes an exterior surface 145. The exterior surface 145 may form an angle 150 relative to the axis 155 through the base portion 125. In at least one example embodiment, the angle 150 may be greater than or equal to about 45°.

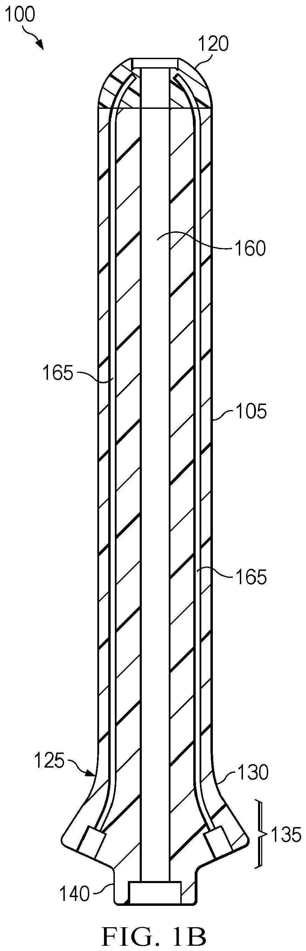

FIG. 1B is a section view of the applicator 100 of FIG. 1A along line I-I according to at least one example embodiment.

In at least one example embodiment, the applicator 100 includes a primary channel 160 and one or more secondary channels 165. The primary channel 160 may extend through a center of the applicator 100 through the base portion 125, the shaft 105, and the cap 120. The one or more secondary channels 165 may circumscribe the primary channel 160. For example, the one or more secondary channel 165 may be equidistantly spaced apart about the primary channel 160. In at least one example embodiment, the one or more secondary channel 165 extend from the second section 135 of the base portion 125 (as shown in FIGS. 1A-1B), the shaft 105, and at least partially through the cap 120. The one or more secondary channels 165 may comprise two to eight channels. In at least one example embodiment, the one or more secondary channels 165 comprises six channels.

In at least one example embodiment, the shaft 105, the cap 120, and/or the base portion 125 of the applicator 100 may comprise a resin or a plastic material. Such materials allow the applicator 100 to be safe in magnetic resonance environments ("MR-Safe"). For example, the materials will not interact with magnetic imaging, such as magnetic resonance imaging ("MRI"). In at least one example embodiment, the shaft 105, the cap 120, and/or the base portion 125 of the applicator 100 may comprise transparent, non-transparent, or a combination of both transparent and non-transparent materials. Transparent materials allow sterilization, cleaning, and disinfection of the application 100 to be checked visually. In at least one example embodiment, surfaces of or materials forming the applicator 100 may also be provided with antibacterial additives such as copper, silver, and/or graphene.

FIG. 2 is a top perspective view of the shaft 105 of the applicator 100 of FIG. 1A according to at least one example embodiment.

In at least one example embodiment, the first end 110 of the shaft 105 includes a top surface 200. The top surface 200 defines a first groove 205 and a second groove 210. In at least one example embodiment, the first groove 205 circumscribes a first central channel 215 extending through the shaft 105 from the first end 110 to the second end 115. The first central channel 215 may form at least a portion of the primary channel 160, as shown in FIG. 1B. In at least one example embodiment, the second groove 210 circumscribes the first groove 205. For example, the second groove 210 may be positioned adjacent an exterior surface of the shaft 105.

In at least one example embodiment, the top surface 200 of the shaft 105 defines one or more holes 220. The one or more holes 220 may be positioned between the first groove 205 and the second groove 210. In at least one example embodiment, the one or more holes 220 comprises three holes. The one or more holes 220 may be spaced equidistantly about a circumference of the top surface 200 of the shaft 105.

In at least one example embodiment, the shaft 105 may include a first plurality of lumens 225 extending from the first end 110 to the second end 115. The first plurality of lumens 225 may form at least a portion of the one or more secondary channels 165 extending through the shaft 105, as shown in FIG. 1B. In at least one example embodiment, the first plurality of lumens 225 may be positioned between the first central channel 215 and the exterior surface of the shaft 105. For example, the first plurality of lumens 225 may be positioned between the first groove 205 and the second groove 210. The first plurality of lumens 225 may also be aligned with the one or more holes 220 in some example embodiments. In at least one example embodiment, the first plurality of lumens 225 may be positioned circumferentially about the first central channel 215. The first plurality of lumens 225 may be equidistantly spaced about the first central channel 215. The first plurality of lumens 225 may comprise two to eight lumens. In at least one example embodiment, the first plurality of lumens 225 comprises six lumens.

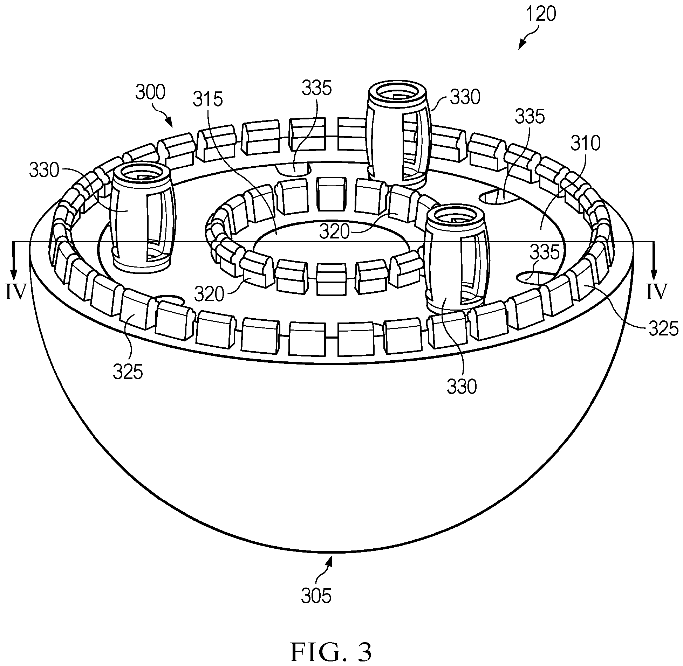

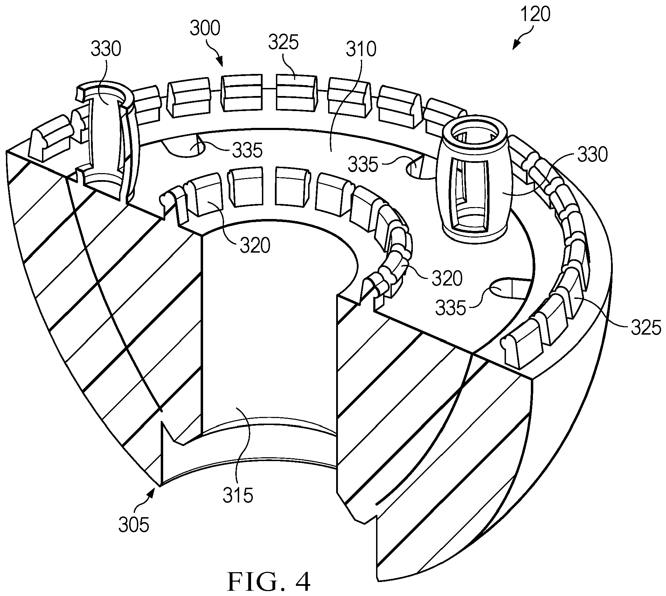

FIG. 3 is a front perspective view of the cap 120 of the applicator 100 of FIG. 1A according to at least one example embodiment. FIG. 4 is a sectional view of the cap 120 of FIG. 3 along line IV-IV according to at least one example embodiment.

In at least one example embodiment, the cap 120 includes a first side 300 and a second side 305. The first side 300 of the cap 120 includes a mating surface 310 configured to be coupled to the top surface 200 of the shaft 105. In at least one example embodiment, the cap 120 defines a second central channel 315 through a center of the cap 120 and extending from the first side 300 to the second side 305 (as shown in FIG. 4). The second central channel 315 is configured to be coupled to the first central channel 215 of the shaft 105. For example, the second central channel 315 may form at least a portion of the primary channel 160 extending through the cap 120, as shown in FIG. 1B.

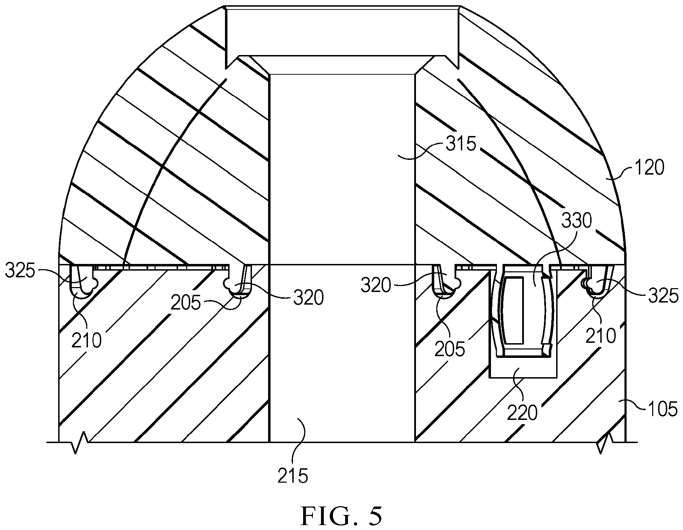

In at least one example embodiment, the cap 120 includes a first plurality of projections 320 and a second plurality of projections 325 extending from the mating surface 310. The first plurality of projections 320 may circumscribe the second central channel 315. The second plurality of projections 325 may circumscribe the first plurality of projections 320. In at least one example embodiment, the second plurality of projections 325 may be positioned adjacent an outer perimeter of the first side 300 of the cap 120. In at least one example embodiment, the first plurality of projections 320 is configured to engage the first groove 205 of the shaft 105 and the second plurality of projections 325 is configured to engage the second groove 210 such that the cap 120 is secured to the first end 110 of the shaft 105, as shown in FIGS. 5-6.

In at least one example embodiment, the cap 120 includes one or more positioning elements 330 extending from the mating surface 310. The one or more positioning elements 330 may be positioned between the first plurality of projections 320 and the second plurality of projections 325. The one or more holes 220 in the top surface 200 of the shaft 105 are configured to receive the one or more positioning elements 330 (as shown in FIG. 5). The one or more positioning elements 330 are configured to align the cap 120 with the shaft 105 such that the cap 120 is properly positioned and secured to the shaft 105. For example, the one or more positioning elements 330 are configured to be inserted into the one or more holes 220 of the shaft 105, as shown in FIGS. 5-6. In at least one example embodiment, the one or more positioning elements 330 may comprise three positioning elements. The one or more positioning elements 330 may be equidistantly spaced about a circumference of the cap 120. In at least one example embodiment, the one or more positioning elements 330 may comprise a flexible material. The flexible material may enable the one or more positioning elements 330 to be inserted into the one or more holes 220 of the shaft 105 by a snap-fit and/or a form-fit.

In at least one example embodiment, the cap 120 defines a plurality of holes 335 extending through at least a portion of the cap 120 from the first side 300 toward the second side 305. The plurality of holes 335 may be positioned between the first plurality of projections 320 and the second plurality of projections 325. The plurality of holes 335 may also be positioned circumferentially about the second central channel 315. The plurality of holes 335 are configured to be coupled to the first plurality of lumens 225 of the shaft 105. For example, the plurality of holes 335 may form at least a portion of the one or more secondary channels 165 extending into the cap 120, as shown in FIG. 1B. In at least one example embodiment, the plurality of holes 335 may comprise two to eight holes. In at least one example embodiment, the plurality of holes may comprise six holes.

FIG. 5 is a detailed interior view of the shaft 105 and the cap 120 of the applicator 100 of FIG. 1A according to at least one example embodiment.

In at least one example embodiment, the first groove 205 and the second groove 210 of the shaft 105 are configured to receive and engage the first plurality of projections 320 and the second plurality of projections 325 of the cap 120, respectively. In at least one example embodiment, a resin may be applied between the shaft 105 and the cap 120. For example, the resin may be applied to the top surface 200 of the shaft 105 and/or to the mating surface 310 of the cap 120. The resin may also be introduced into the first groove 205, the second groove 210, and/or the one or more holes 220 of the shaft. After applying the resin and connecting the cap 120 to the shaft 104, the resin is cured such that the cap 120 is secured to the shaft 105.

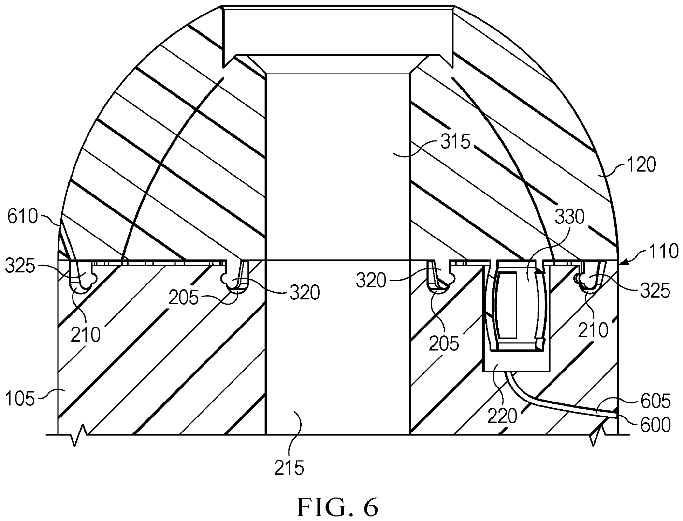

FIG. 6 is a detailed interior view of the shaft 105 and the cap 120 of the applicator 100 of FIG. 1A according to at least one example embodiment.

In at least one example embodiment, a resin may be introduced between the cap 120 and the shaft 105 after the cap 102 and the shaft 105 are already connected. For example, the shaft 105 may include an inlet 600 in a side of the shaft adjacent the first end 110 of the shaft 105. The shaft 105 may also include a channel 605 fluidly coupling the inlet 600 with one or more of the holes 220. Resin may be introduced to the one or more holes 220 through the inlet 600 and the channel 605. The resin may also flow from the one or more holes 220 along the top surface 200 of the shaft and the mating surface 310 of the cap 120, and into the first groove 205 and the second groove 210.

In at least one example embodiment, the shaft 105 also includes an outlet 610 opposite the inlet 600. For example, the outlet 610 may be fluidly coupled to the second groove 210 on a side of the shaft 105 opposite the inlet 600. The outlet 610 may also be positioned above the inlet 600 and the channel 605. For example, the outlet 610 may be positioned closer to the first end 110 of the shaft 105 than the inlet 600. In another example embodiment, the outlet 610 may be disposed in the cap 120 adjacent the second plurality of projections 325 and opposite the inlet 600. The outlet 610 may be configured to allow air and excess resin to flow out as resin is introduced through the inlet 600.

FIG. 7 is a top perspective view of the base portion 125 of the applicator 100 of FIG. 1A according to at least one example embodiment.

In at least one example embodiment, the first section 130 of the base portion 125 includes a mating surface 700. The mating surface 700 may be similar or analogous to the top surface 200 of the shaft 105, as shown in FIG. 2. The mating surface 700 defines a first groove 705 and a second groove 710. In at least one example embodiment, the first groove 705 circumscribes a third central channel 715. The third central channel 715 extends through the first section 130, the second section 135, and the third section 140 of the base portion 125 and may form at least a portion of the primary channel 160, as discussed above with respect to FIG. 1B. In at least one example embodiment, the second groove 710 is adjacent an outer perimeter of the base portion 125 and circumscribes the first groove 705.

In at least one example embodiment, the mating surface 700 of the base portion 125 defines one or more holes 720. The one or more holes 720 may extend from the mating surface 700 at least partially into the first section 130 of the base portion 125. In at least one example embodiment, the one or more holes 720 comprises three holes. In at least one example embodiment, the one or more holes 720 may be positioned between the first groove 705 and the second groove 710 and equidistantly spaced apart about the third central channel 715.

In at least one example embodiment, the base portion 125 includes a second plurality of lumens 725. The second plurality of lumens 725 may extend through the first section 130 and the second section 135 of the base portion 125 and form at least a portion of the one or more secondary channels 165, as discussed above with respect to FIG. 1B. In at least one example embodiment, the second plurality of lumens 725 may be positioned between the first groove 705 and the second groove 710. The second plurality of lumens 725 may also be aligned with the one or more holes 720 in some embodiments. In at least one example embodiment, the second plurality of lumens 725 may be equidistantly spaced apart about the third central channel 715. In at least one example embodiment, the second plurality of lumens 725 comprises two to eight lumens. In at least one example embodiment, the second plurality of lumens 725 comprises six lumens.

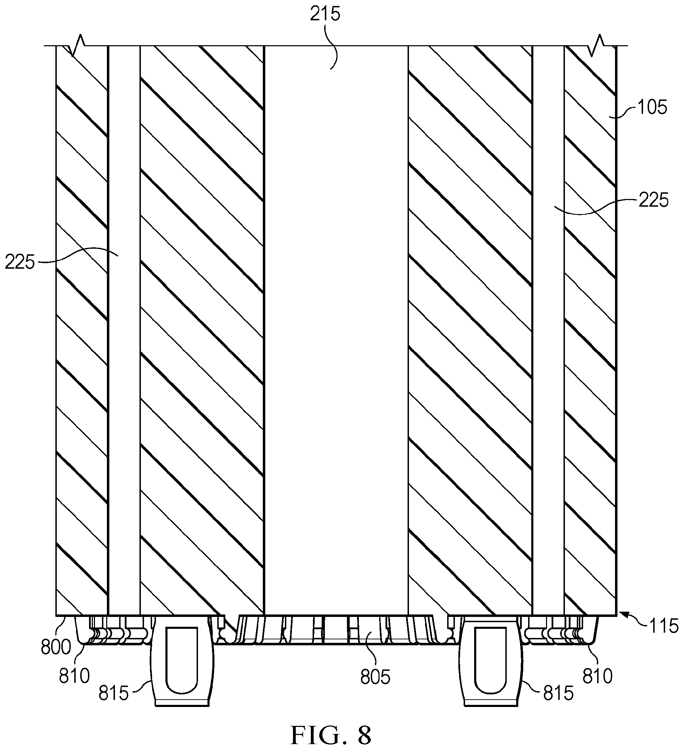

FIG. 8 is a detailed, section view of the second end 115 of the shaft 105 of the applicator 100 of FIG. 1A according to at least one example embodiment.

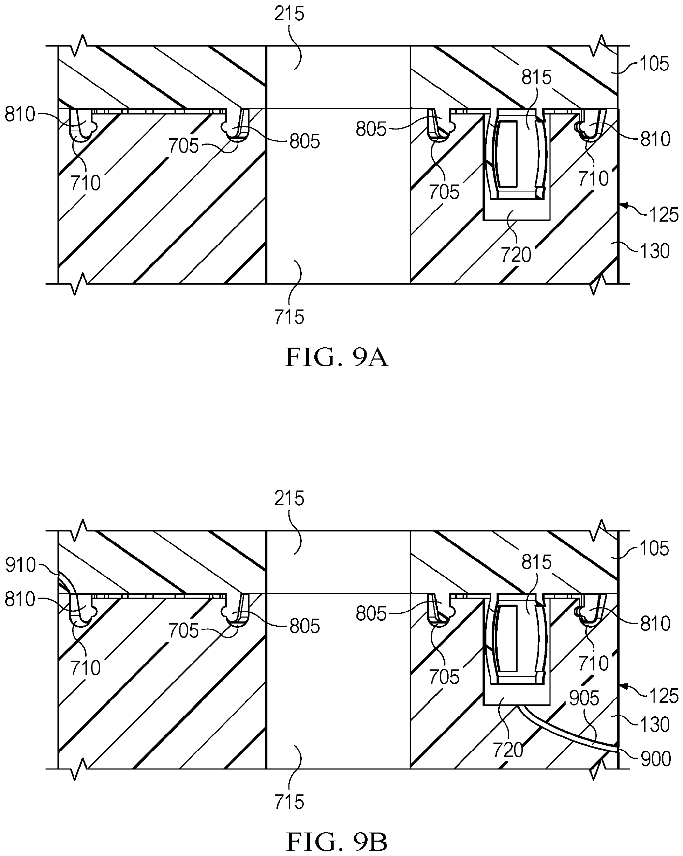

In at least one example embodiment, the second end 115 of the shaft 105 includes a bottom surface 800. The bottom surface 800 of the shaft 105 may be similar or analogous to the mating surface 310 of the cap 120, discussed above with respect to FIG. 3. The mating surface 700 of the base portion 125 is configured to be coupled to the bottom surface 800 of the shaft 105, as will be discussed in FIG. 9, below.

In at least one example embodiment, the shaft 105 includes a first plurality of projections 805 and a second plurality of projections 810 extending from the bottom surface 800. The first plurality of projections 805 may circumscribe the first central channel 215. The second plurality of projections 810 may circumscribe the first plurality of projections 805. In at least one example embodiment, the second plurality of projections 810 may be positioned adjacent an outer perimeter of the shaft 105. For example, the second plurality of projections 810 may be between the outer perimeter of the shaft 105 and the first plurality of lumens 225. In at least one example embodiment, the first plurality of projections 805 is configured to engage the first groove 705 of the base portion 125 and the second plurality of projections 810 is configured to engage the second groove 710 of the base portion 125 such that the base portion 125 is secured to the second end 115 of the shaft 105.

In at least one example embodiment, the shaft 105 includes one or more positioning elements 815 extending from the bottom surface 800. The one or more positioning elements 815 may be positioned between the first plurality of projections 805 and the second plurality of projections 810. In at least one example embodiment, the one or more holes 720 in the mating surface 700 of the base portion 125 are configured to receive the one or more positioning elements 815. The one or more positioning elements 815 are configured to align the shaft 105 and the base portion 125 such that the base portion 125 is properly positioned and secured to the shaft 105. In at least one example embodiment, the one or more positioning elements 815 may comprise three positioning elements. In at least one example embodiment, the one or more positioning elements 815 are spaced equidistantly about the first central channel 215. In at least one example embodiment, the one or more positioning elements 815 may comprise a flexible material. The flexible material may enable the one or more positioning elements 815 to be inserted into the one or more holes 720 of the base portion 125 by a snap-fit and/or a form-fit.

FIG. 9A is a detailed section view of the shaft 105 and the base portion 125 of the applicator 100 of FIG. 1A according to at least one example embodiment.

In at least one example embodiment, the first groove 705 and the second groove 710 of the base portion 125 are configured to receive and engage the first plurality of projections 805 and the second plurality of projections 810 of the shaft 105, respectively. In at least one example embodiment, a resin may be applied between the base portion 125 and the shaft 105. For example, the resin may be applied to the mating surface 700 of the base portion 125 and/or the bottom surface 800 of the shaft 105 before connecting the base portion 125 and the shaft 105. The resin may also be introduced into the first groove 705, the second groove 710, and/or the one or more holes 720. After the resin is applied and the base portion 125 is connected to the shaft 105, the resin may be cured such that the base portion 125 is secured to the shaft 105.

FIG. 9B is a detailed section view of the shaft and the base portion of the applicator of FIG. 1A according to at least one example embodiment.

In at least one example embodiment, the resin may be introduced between the shaft 105 and the base portion 125 after the base portion 125 is coupled to the shaft 105. For example, the base portion 125 may include an inlet 900 for introducing the resin. The inlet may also include a channel 905 configured to fluidly couple the inlet 900 to the one or more holes 720. The shaft 105 may also include an outlet 910 opposite the inlet for allowing air and excess resin to flow out from between the shaft 105 and the base portion 125. In at least one example embodiment, the outlet 910 is fluidly coupled to the second groove 710. The inlet 900, the channel 905, and the outlet 910 may be similar or analogous to the inlet 600, the channel 605, and the outlet 610, as discussed above with respect to FIG. 6. For example, the resin may be introduced to the one or more holes 720 of the base portion 125 through the inlet 900 and the channel 905. The resin may also flow from the one or more holes 720 along the mating surface 700 of the base portion and the bottom surface 800 of the shaft 105, and into the first groove 705 and the second groove 710. The outlet 910 may be configured to allow air and excess resin to flow out from between the base portion 125 and the shaft 105.

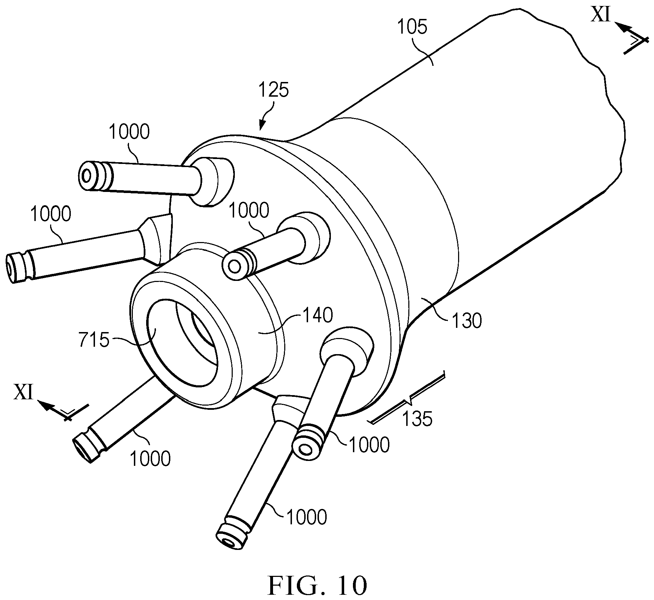

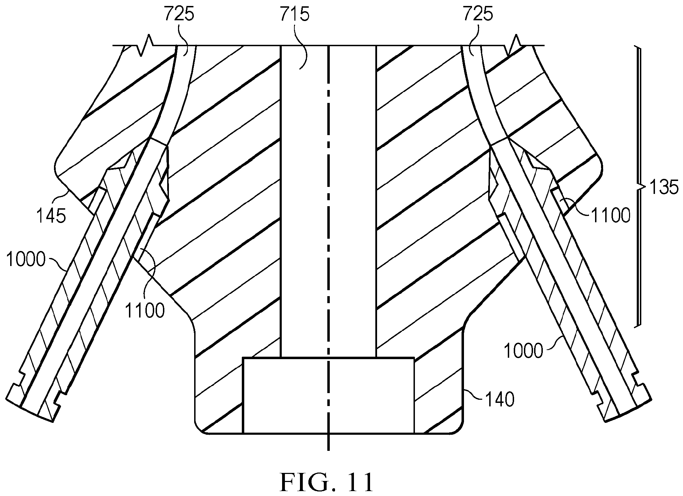

FIG. 10 is a detailed perspective view of the base portion 125 of FIG. 1A according to at least one example embodiment. FIG. 11 is a section view of the base portion 125 of FIG. 11 along line XI-XI according to at least one example embodiment.

In at least one example embodiment, a plurality of connectors 1000 may be configured to be at least partially inserted into the second plurality of lumens 725 of the base portion 125. For example, the exterior surface 145 of the second section 135 of the base portion 125 may define a plurality of openings 1100. The plurality of openings may be coupled to the second plurality of lumens 725 and configured to receive the plurality of connectors 1000. The plurality of connectors 1000 may comprise the same material as the shaft 105, the cap 120, and/or the base portion 125 of the applicator 100.

In at least one example embodiment, the plurality of connectors 1000 may be coupled to the base portion 125 with a resin. For example, a resin may be applied to each of the plurality of openings 1100 and/or the second plurality of lumens 725 before inserting the plurality of connectors 1000. After inserting the plurality of connectors 1000 into the plurality of openings 1100, the resin may then be cured such that the plurality of connectors 1000 are secured to the base portion 125. In at least one example embodiment, the resin applied to the plurality of openings 1100 and the plurality of connectors 1000 may be cured at the same time the resin applied between the shaft 105 and the base portion 125 and/or the resin applied between the shaft 105 and the cap 120 is cured.

In at least one example embodiment, the plurality of connectors 1000 are configured to be coupled to the second plurality of lumens 725 of the base portion 125, the first plurality of lumens 225 of the shaft 105, and the plurality of holes 335 of the cap 120. For example, the plurality of connectors 1000 may be configured to be coupled to the one or more secondary channels 165 of the applicator 100, as shown in FIG. 1B.

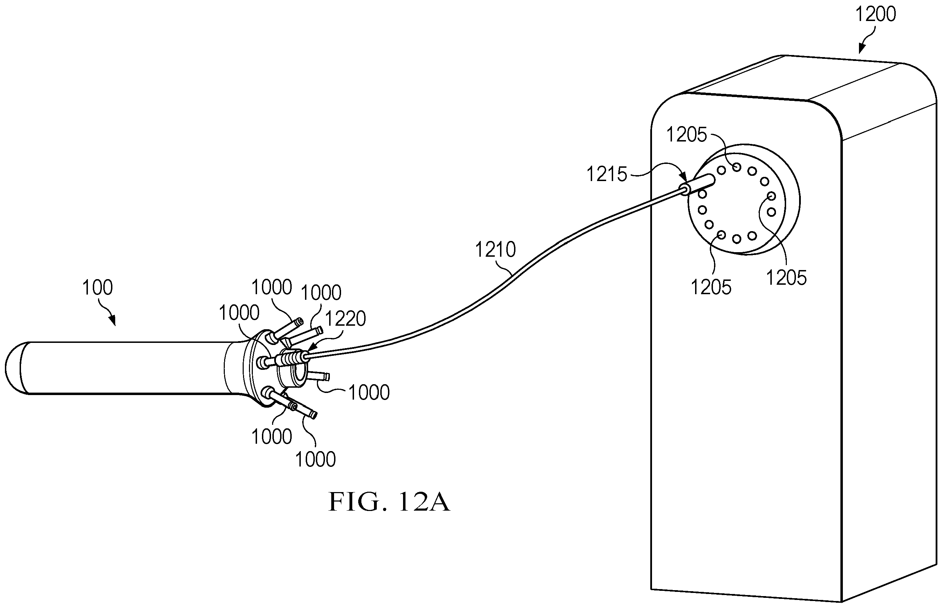

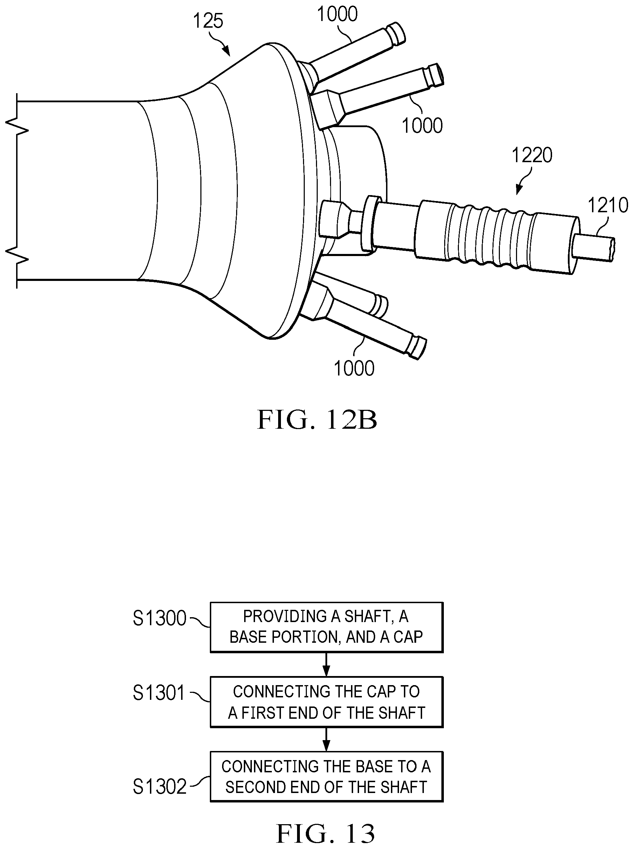

FIG. 12A is a perspective view of the applicator of FIG. 1A and a source of radiation according to at least one example embodiment. FIG. 12B is a detailed view of the applicator of FIG. 12A according to at least one example embodiment.

In at least one example embodiment, the plurality of connectors 1000 may be coupled to a source of radiation 1200. The source of radiation 1200 may include one or more ports 1205 configured to receive one or more wires or cables 1210. For example, a first end 1215 of the cable 1210 may be coupled to one or the ports 1205 and a second end 1220 of the cable 1210 may be coupled to one of the connectors 1000 (as shown in FIG. 12B). The applicator 100 may be configured to deliver radiation from the source of radiation 1200 to a target location. For example, radiation may be supplied from the source of radiation 1200 through the one or more cables 1210, the plurality of connectors 1000, and the one or more secondary channels 165 of the applicator 100.

FIG. 13 is a flowchart depicting a method of manufacturing an applicator according to at least one example embodiment.

As shown in FIG. 13, in at least one example embodiment, the method generally includes providing the shaft 105, the base portion 125, and the cap 120 at S1300; connecting the cap 120 to the first end 110 of the shaft 105 at S1301; and connecting the base portion 125 to the second end 115 of the shaft 105 at S1302. Each of these steps is described in greater detail below.

At S1300, in at least one example embodiment, the method includes providing the shaft 105, the base portion 125, and the cap 120. Providing the shaft 105, the cap 120, and the base portion 125 includes forming the shaft 105, the cap 120, and the base portion 125 by additive manufacturing. In at least one example embodiment, the shaft 105 and the base portion 125 may be a single unitary piece that may be formed by additive manufacturing. Additive manufacturing allows the shaft 105, the cap 120, and/or the base portion 125 to be manufactured modularly. Additive manufacturing also allows the applicator 100 to be customizable. For example, the number of channels and the size of the channel, such as the primary channel 160 and the one or more secondary channels 165 may be customized to suit particular treatment or patient needs. Additionally or alternatively, the length, diameter, and/or angle of the applicator 100 may also be customized to suit particular treatment or patient needs. Producing the applicator 100 by additive manufacturing may also reduce costs of manufacturing.

In at least one example embodiment, the method further includes cleaning the shaft 105, the cap 120, and the base portion 125. For example, the shaft 105, the cap 120, and the base portion 125 may be cleaned before connecting the cap 120 to the shaft 105 and before connecting the base portion 125 to the shaft 105.

At S1301, in at least one example embodiment, the method includes connecting the cap 120 to the first end 110 of the shaft 105. Connecting the cap 120 to the shaft 105 may include engaging the first plurality of projections 320 of the cap 120 with the first groove 205 of the shaft 105 and/or engaging the second plurality of projections 325 of the cap 120 with the second groove 210 of the shaft 105. In at least one example embodiment, the connecting the cap 120 to the shaft 105 includes inserting the one or more positioning elements 330 of the cap 120 into the one or more holes 220 of the shaft 105.

As described above in relation to FIGS. 5-6, connecting the cap 120 to the shaft 105 may also include applying a resin between the shaft 105 and the cap 120. In at least one example embodiment, the method includes forming the inlet 600 and the channel 605 in a side of the shaft 105 adjacent the first end 110 and forming the outlet 610 in another side of the shaft 105 opposite the inlet 600. In such example embodiment, the method includes applying the resin between the shaft 105 and the cap 120 through the inlet 600.

At S1302, in at least one example embodiment, the method includes connecting the base portion 125 to the second end 115 of the shaft 105. Connecting the base portion 125 to the shaft 105 may include engaging the first plurality of projections 805 of the shaft 105 with the first groove705 of the base portion and/or engaging the second plurality of projections 810 of the shaft 105 with the second groove 710 of the base portion 125. In at least one example embodiment, connecting the base portion 125 to the shaft 105 may also include inserting the one or more positioning elements 815 of the shaft 105 into the one or more holes 720 of the base portion 125. The method may further include applying a resin between the shaft 105 and the base portion 125. In at least one example embodiment, the resin is applied before connecting the base portion 125 to the shaft 105. In other example embodiments, the resin may be applied after the base portion 125 is connected to the shaft 105. For example, the shaft 105 and/or the base portion 125 may include another inlet, channel, and outlet at the second end 115 of the shaft 105, similar to the inlet 600, the channel 605, and the outlet 610 discussed above with respect to FIG. 6.

In at least one example embodiment, the method includes coupling the plurality of connectors 1000 to the base portion 125. For example, the method may include inserting the plurality of connectors 1000 into the plurality of openings 1100 of the base portion 125. The method may further include applying a resin between the base portion 125 and the plurality of connectors 1000, such as introducing the resin into the plurality of openings 1100.

In at least one example embodiment, the method further includes curing the resin such that the cap 120 and the base portion 125 are secured to the shaft 105 and the applicator 100 is formed. The method may further include cleaning the applicator 100 such that any excess resin may be removed and the applicator 100 may be sanitized.

## Claims

1. A method of manufacturing a brachytherapy applicator (100), comprising:
providing a shaft (105), the shaft having a first end (110) and a second end (115) opposite the first end (110);
providing a cap (120);
providing a base portion (125); and
forming an applicator (100), the forming including,
connecting the cap (120) to the first end (110) of the shaft, and
connecting the base portion (125) to the second end (115) of the shaft,
wherein optionally the shaft (105), the cap (120), and the base portion (125) comprise a resin or a plastic material;
wherein the first end (110) of the shaft comprises a top surface (200), the top surface defining a first groove (205) and a second groove (210), the second groove (210) circumscribing the first groove (205), and the cap (120) includes a mating surface (310) configured to be coupled to the top surface (200) of the shaft (105);
a first plurality of projections (320) extending from the mating surface (310); and
a second plurality of projections (325) extending from the mating surface (310) and circumscribing the first plurality of projections (320); and
wherein the connecting the cap to the first end of the shaft comprises:
engaging the first plurality of projections (320) of the cap (120) with the first groove (205) of the shaft (105); and
engaging the second plurality of projections (325) of the cap (120) with the second groove (210) of the shaft (105)

2. The method of claim 1, wherein:
the providing the shaft includes forming the shaft (105) by additive manufacturing;
the providing a cap includes forming the cap (120) by additive manufacturing; and
the providing the base portion includes forming the base portion (125) by additive manufacturing and optionally
wherein the forming an applicator further comprises:
applying a resin between the shaft (105) and the cap (120);
applying the resin between the shaft (150) and the base portion (125); and
curing the resin.

3. The method of any one of claims 1 to 2, wherein:
the first end of (110) the shaft comprises a top surface (200), the top surface defining one or more holes (220); and
the cap (120) includes a mating surface configured to be coupled to the top surface (200) of the shaft and one or more positioning elements (330) extending from the mating surface (310) and optionally the connecting the cap (120) to the first end (110) of the shaft includes inserting the one or more positioning elements (330) of the cap into the one or more holes (220) of the shaft (105).

4. The method of any one of the preceding claims, further comprising:
forming an inlet (600) in a side of the shaft adjacent the first end (110) of the shaft (105);
forming an outlet (610) in another side of the shaft (105); and
applying a resin between the shaft (105) and the cap (120) through the inlet (600).

5. The method of any one of the preceding claims, wherein the forming the applicator further comprises:
coupling a plurality of connectors (1000) to the base portion (125); and
applying a resin between the connectors (1000) and the base portion (125).

6. The method of any one of the preceding claims, wherein:
the shaft (105) includes a first central channel (215) extending from the first end (110) to the second end (115) and a first plurality of lumens (225) extending from the first end (110) to the second end (115), the first plurality of lumens (225) positioned circumferentially about the first central channel (215);
the cap (120) includes a second central channel (315) extending through a center of the cap (120) and a plurality of holes (220) extending at least partially through the cap (120), the second central channel (315) configured to be coupled to the first central channel (215), the plurality of holes (220) positioned circumferentially about the second central channel (315) and configured to be coupled to the first plurality of lumens (225); and
the base portion (125) includes a third central channel (715) extending through a center of the base portion (125) and a second plurality of lumens (725) extending through the base portion (125), the third central channel (715) configured to be coupled to the first central channel (215), the second plurality of lumens (725) positioned circumferentially about the third central channel (715) and configured to be coupled to the first plurality of lumens (225).

7. The method of any one of the preceding claims, further comprising cleaning the shaft (105), the base portion (125), and the cap (120) before the forming the applicator (100).

8. A brachytherapy applicator (100) for insertion into a body cavity, comprising:
a shaft (105) having a first end (110) and a second end (115) opposite the first end, the shaft (105) including a top surface (200) adjacent the first end of the shaft, the top surface (200) defining a first groove (205) and a second groove (210), the second groove (210) circumscribing the first groove (205); and
a cap (120) configured to be coupled to the first end (110) of the shaft (105), the cap (120) including,
a mating surface (310) configured to be coupled to the top surface (200) of the shaft (105),
a first plurality of projections (320) extending from the mating surface (310), the first plurality of projections (320) configured to engage the first groove (205), and
a second plurality of projections (325) extending from the mating surface (310) and circumscribing the first plurality of projections (320), the second plurality of projections (325) configured to engage the second groove (210).

9. The brachytherapy applicator of claim 8, wherein:
the cap (120) comprises one or more positioning elements (330) extending from the mating surface (310); and
the top surface (200) of the shaft (105) defines one or more holes (220) configured to receive the one or more positioning elements (330).

10. The brachytherapy applicator of claim 8 or 9, wherein:
the shaft (105) includes a first central channel (215) extending from the first end (110) to the second end (115) and a first plurality of lumens (225) extending from the first end (110) to the second end (115), the first plurality of lumens (225) positioned circumferentially about the first central channel (215); and
the cap (120) includes a second central channel (315) extending through a center of the cap (120) and a plurality of holes (335) extending at least partially through the cap (120), the second central channel (315) configured to be coupled to the first central channel (215), the plurality of holes (335) positioned circumferentially about the second central channel (315) and configured to be coupled to the first plurality of lumens(225).

11. The brachytherapy applicator of any one of claims 8-10, wherein the second end of the shaft comprises a base portion (125), the base portion including,
a first section (130) configured to be coupled to the second end (115) of the shaft;
a second section (135) including an exterior surface (145), the exterior surface (145) at an angle (150) relative to a horizontal axis (155) through the base portion (125);
a third section (140), the second section (135) between the first section (130) and the third section (140);
a third central channel (715) extending through the first section (130), the second section (135), and the third section (140); and
a second plurality of lumens (725) extending through the first section (130) and the second section (135) of the base portion (125), and optionally the angle is greater than or equal to 45°.

12. The brachytherapy applicator of claim 10 or 11, further comprising a plurality of connectors (1000) configured to be at least partially inserted into the first plurality of lumens (725) of the third section (140) of the base portion (125).

13. The brachytherapy applicator of claim 12, further comprising a source of radiation (1200) configured to be coupled to the plurality of connectors (1000) and to deliver radiation to a target location.

## Patentansprüche

1. Verfahren zum Herstellen eines Brachytherapieapplikators (100), umfassend:
Bereitstellen eines Schafts (105), wobei der Schaft ein erstes Ende (110) und ein zweites Ende (115) gegenüber dem ersten Ende (110) aufweist;
Bereitstellen einer Kappe (120);
Bereitstellen eines Basisabschnitts (125); und
Ausbilden eines Applikators (100), wobei das Ausbilden Folgendes beinhaltet:
Verbinden der Kappe (120) mit dem ersten Ende (110) des Schafts und
Verbinden des Basisabschnitts (125) mit dem zweiten Ende (115) des Schafts,
wobei optional der Schaft (105), die Kappe (120) und der Basisabschnitt (125) ein Harz oder ein Kunststoffmaterial umfassen;
wobei das erste Ende (110) des Schafts eine Oberseite (200) umfasst, wobei die Oberseite eine erste Nut (205) und eine zweite Nut (210) definiert, wobei die zweite Nut (210) die erste Nut (205) abgrenzt und die Kappe (120) eine Gegenfläche (310) beinhaltet, die dazu konfiguriert ist, an die Oberseite (200) des Schafts (105) gekoppelt zu werden;
wobei sich eine erste Vielzahl von Vorsprüngen (320) von der Gegenfläche (310) erstreckt; und
wobei sich eine zweite Vielzahl von Vorsprüngen (325) von der Gegenfläche (310) erstreckt und die erste Vielzahl von Vorsprüngen (320) abgrenzt; und
wobei das Verbinden der Kappe mit dem ersten Ende des Schafts Folgendes umfasst:
Eingreifen der ersten Vielzahl von Vorsprüngen (320) der Kappe (120) in die erste Nut (205) des Schafts (105); und
Eingreifen der zweiten Vielzahl von Vorsprüngen (325) der Kappe (120) in die zweite Nut (210) des Schafts (105).

2. Verfahren nach Anspruch 1, wobei:
das Bereitstellen des Schafts Ausbilden des Schafts (105) durch additive Fertigung beinhaltet;
das Bereitstellen einer Kappe Ausbilden der Kappe (120) durch additive Fertigung beinhaltet; und
das Bereitstellen des Basisabschnitts Ausbilden des Basisabschnitts (125) durch additive Fertigung beinhaltet und wobei optional
das Ausbilden eines Applikators ferner Folgendes umfasst:
Aufbringen eines Harzes zwischen dem Schaft (105) und der Kappe (120);
Aufbringen des Harzes zwischen dem Schaft (150) und dem Basisabschnitt (125); und
Aushärten des Harzes.

3. Verfahren nach einem der Ansprüche 1 bis 2, wobei:
das erste Ende (110) des Schafts eine Oberseite (200) umfasst, wobei die Oberseite ein oder mehrere Löcher (220) definiert; und
die Kappe (120) eine Gegenfläche, die dazu konfiguriert ist, an die Oberseite (200) des Schafts gekoppelt zu werden, und ein oder mehrere Positionierungselemente (330) beinhaltet, die sich von der Gegenfläche (310) erstrecken, und wobei optional das Verbinden der Kappe (120) mit dem ersten Ende (110) des Schafts Einführen des einen oder der mehreren Positionierungselemente (330) der Kappe in das eine oder die mehreren Löcher (220) des Schafts (105) beinhaltet.

4. Verfahren nach einem der vorhergehenden Ansprüche, ferner umfassend:
Ausbilden eines Einlasses (600) in einer Seite des Schafts benachbart zu dem ersten Ende (110) des Schafts (105);
Ausbilden eines Auslasses (610) in einer anderen Seite des Schafts (105); und
Aufbringen eines Harzes zwischen dem Schaft (105) und der Kappe (120) durch den Einlass (600).

5. Verfahren nach einem der vorhergehenden Ansprüche, wobei das Ausbilden des Applikators ferner Folgendes umfasst:
Koppeln einer Vielzahl von Verbindern (1000) an den Basisabschnitt (125); und
Aufbringen eines Harzes zwischen den Verbindern (1000) und dem Basisabschnitt (125).

6. Verfahren nach einem der vorhergehenden Ansprüche, wobei:
der Schaft (105) einen ersten zentralen Kanal (215), der sich von dem ersten Ende (110) zu dem zweiten Ende (115) erstreckt, und eine erste Vielzahl von Lumen (225), die sich von dem ersten Ende (110) zu dem zweiten Ende (115) erstreckt, beinhaltet, wobei die erste Vielzahl von Lumen (225) in Umfangsrichtung um den ersten zentralen Kanal (215) positioniert ist;
die Kappe (120) einen zweiten zentralen Kanal (315), der sich durch ein Zentrum der Kappe (120) erstreckt, und eine Vielzahl von Löchern (220), die sich zumindest teilweise durch die Kappe (120) erstreckt, beinhaltet, wobei der zweite zentrale Kanal (315) dazu konfiguriert ist, an den ersten zentralen Kanal (215) gekoppelt zu werden, wobei die Vielzahl von Löchern (220) in Umfangsrichtung um den zweiten zentralen Kanal (315) positioniert und dazu konfiguriert ist, an die erste Vielzahl von Lumen (225) gekoppelt zu werden; und
der Basisabschnitt (125) einen dritten zentralen Kanal (715), der sich durch ein Zentrum des Basisabschnitts (125) erstreckt, und eine zweite Vielzahl von Lumen (725), die sich durch den Basisabschnitt (125) erstreckt, beinhaltet, wobei der dritte zentrale Kanal (715) dazu konfiguriert ist, an den ersten zentralen Kanal (215) gekoppelt zu werden, wobei die zweite Vielzahl von Lumen (725) in Umfangsrichtung um den dritten zentralen Kanal (715) positioniert und dazu konfiguriert ist, an die erste Vielzahl von Lumen (225) gekoppelt zu werden.

7. Verfahren nach einem der vorhergehenden Ansprüche, ferner umfassend Reinigen des Schafts (105), des Basisabschnitts (125) und der Kappe (120) vor dem Ausbilden des Applikators (100).

8. Brachytherapieapplikator (100) zum Einführen in einen Körperhohlraum, umfassend:
einen Schaft (105), der ein erstes Ende (110) und ein zweites Ende (115) gegenüber dem ersten Ende aufweist, wobei der Schaft (105) eine Oberseite (200) benachbart zu dem ersten Ende des Schafts beinhaltet, wobei die Oberseite (200) eine erste Nut (205) und eine zweite Nut (210) definiert, wobei die zweite Nut (210) die erste Nut (205) abgrenzt; und
eine Kappe (120), die dazu konfiguriert ist, an das erste Ende (110) des Schafts (105) gekoppelt zu werden, wobei die Kappe (120) Folgendes beinhaltet,
eine Gegenfläche (310), die dazu konfiguriert ist, an die Oberseite (200) des Schafts (105) gekoppelt zu werden,
eine erste Vielzahl von Vorsprüngen (320), die sich von der Gegenfläche (310) erstreckt, wobei die erste Vielzahl von Vorsprüngen (320) dazu konfiguriert ist, in die erste Nut (205) einzugreifen, und
eine zweite Vielzahl von Vorsprüngen (325), die sich von der Gegenfläche (310) erstreckt und die erste Vielzahl von Vorsprüngen (320) abgrenzt, wobei die zweite Vielzahl von Vorsprüngen (325) dazu konfiguriert ist, in die zweite Nut (210) einzugreifen.

9. Brachytherapieapplikator nach Anspruch 8, wobei:
die Kappe (120) ein oder mehrere Positionierungselemente (330) umfasst, die sich von der Gegenfläche (310) erstrecken; und
die Oberseite (200) des Schafts (105) ein oder mehrere Löcher (220) definiert, die dazu konfiguriert sind, das eine oder die mehreren Positionierungselemente (330) aufzunehmen.

10. Brachytherapieapplikator nach Anspruch 8 oder 9, wobei:
der Schaft (105) einen ersten zentralen Kanal (215), der sich von dem ersten Ende (110) zu dem zweiten Ende (115) erstreckt, und eine erste Vielzahl von Lumen (225), die sich von dem ersten Ende (110) zu dem zweiten Ende (115) erstreckt, beinhaltet, wobei die erste Vielzahl von Lumen (225) in Umfangsrichtung um den ersten zentralen Kanal (215) positioniert ist; und
die Kappe (120) einen zweiten zentralen Kanal (315), der sich durch ein Zentrum der Kappe (120) erstreckt, und eine Vielzahl von Löchern (335), die sich zumindest teilweise durch die Kappe (120) erstreckt, beinhaltet, wobei der zweite zentrale Kanal (315) dazu konfiguriert ist, an den ersten zentralen Kanal (215) gekoppelt zu werden, wobei die Vielzahl von Löchern (335) in Umfangsrichtung um den zweiten zentralen Kanal (315) positioniert und dazu konfiguriert ist, an die erste Vielzahl von Lumen (225) gekoppelt zu werden; und

11. Brachytherapieapplikator nach einem der Ansprüche 8-10, wobei das zweite Ende des Schafts einen Basisabschnitt (125) umfasst, wobei der Basisabschnitt Folgendes beinhaltet:
einen ersten Bereich (130), der dazu konfiguriert ist, an das zweite Ende (115) des Schafts gekoppelt zu werden;
einen zweiten Bereich (135), der eine Außenfläche (145) beinhaltet, wobei die Außenfläche (145) in einem Winkel (150) relativ zu einer horizontalen Achse (155) durch den Basisabschnitt (125) liegt;
einen dritten Bereich (140), wobei der zweite Bereich (135) zwischen dem ersten Bereich (130) und dem dritten Bereich (140) liegt;
einen dritten zentralen Kanal (715), der sich durch den ersten Bereich (130), den zweiten Bereich (135) und den dritten Bereich (140) erstreckt; und
eine zweite Vielzahl von Lumen (725), die sich durch den ersten Bereich (130) und den zweiten Bereich (135) des Basisabschnitts (125) erstreckt, und wobei optional der Winkel größer oder gleich 45° ist.

12. Brachytherapieapplikator nach Anspruch 10 oder 11, ferner umfassend eine Vielzahl von Verbindern (1000), die dazu konfiguriert ist, zumindest teilweise in die erste Vielzahl von Lumen (725) des dritten Bereichs (140) des Basisabschnitts (125) eingeführt zu werden.

13. Brachytherapieapplikator nach Anspruch 12, ferner umfassend eine Strahlungsquelle (1200), die dazu konfiguriert ist, an die Vielzahl von Verbindern (1000) gekoppelt zu werden und Strahlung an eine Zielstelle abzugeben.

## Revendications

1. Procédé de fabrication d'un applicateur de brachythérapie (100), comprenant :
la fourniture d'une tige (105), la tige présentant une première extrémité (110) et une seconde extrémité (115) opposée à la première extrémité (110) ;
la fourniture d'un capuchon (120) ;
la fourniture d'une partie de base (125) ; et
la formation d'un applicateur (100), la formation comportant,
la connexion du capuchon (120) à la première extrémité (110) de la tige, et
la connexion de la partie de base (125) à la seconde extrémité (115) de la tige,
dans lequel éventuellement la tige (105), le capuchon (120) et la partie de base (125) comprennent une résine ou une matière plastique ;
dans lequel la première extrémité (110) de la tige comprend une surface supérieure (200), la surface supérieure définissant une première rainure (205) et une seconde rainure (210), la seconde rainure (210) circonscrivant la première rainure (205), et le capuchon (120) comporte une surface d'accouplement (310) configurée pour être couplée à la surface supérieure (200) de la tige (105) ;
une première pluralité de saillies (320) se prolongeant à partir de la surface d'accouplement (310) ; et
une seconde pluralité de saillies (325) se prolongeant à partir de la surface d'accouplement (310) et circonscrivant la première pluralité de saillies (320) ; et
dans lequel la connexion du capuchon à la première extrémité de la tige comprend :
la mise en contact de la première pluralité de saillies (320) du capuchon (120) avec la première rainure (205) de la tige (105) ; et
la mise en contact de la seconde pluralité de saillies (325) du capuchon (120) avec la seconde rainure (210) de la tige (105).

2. Procédé selon la revendication 1, dans lequel :
la fourniture de la tige comporte la formation de la tige (105) par fabrication additive ;
la fourniture d'un capuchon comporte la formation du capuchon (120) par fabrication additive ; et
la fourniture de la partie de base comporte la formation de la partie de base (125) par fabrication additive et éventuellement
dans lequel la formation d'un applicateur comprend également :
l'application d'une résine entre la tige (105) et le capuchon (120) ;
l'application de la résine entre la tige (150) et la partie de base (125) ; et
le durcissement de la résine.

3. Procédé selon l'une quelconque des revendications 1 ou 2, dans lequel :
la première extrémité (110) de la tige comprend une surface supérieure (200), la surface supérieure définissant un ou plusieurs trous (220) ; et
le capuchon (120) comporte une surface d'accouplement configurée pour être couplée à la surface supérieure (200) de la tige et un ou plusieurs éléments de positionnement (330) se prolongeant à partir de la surface d'accouplement (310) et éventuellement la connexion du capuchon (120) à la première extrémité (110) de la tige comporte l'insertion des un ou plusieurs éléments de positionnement (330) du capuchon dans les un ou plusieurs trous (220) de la tige (105).

4. Procédé selon l'une quelconque des revendications précédentes, comprenant également :
la formation d'une entrée (600) dans un côté de la tige adjacent à la première extrémité (110) de la tige (105) ;
la formation d'une sortie (610) dans un autre côté de la tige (105) ; et
l'application d'une résine entre la tige (105) et le capuchon (120) à travers l'entrée (600).

5. Procédé selon l'une quelconque des revendications précédentes, dans lequel la formation de l'applicateur comprend également :
l'accouplement d'une pluralité de connecteurs (1000) à la partie de base (125) ; et
l'application d'une résine entre les connecteurs (1000) et la partie de base (125).

6. Procédé selon l'une quelconque des revendications précédentes dans lequel :
la tige (105) comporte un premier canal central (215) se prolongeant de la première extrémité (110) à la seconde extrémité (115) et une première pluralité de lumières (225) se prolongeant de la première extrémité (110) à la seconde extrémité (115), la première pluralité de lumières (225) étant positionnée circonférentiellement autour du premier canal central (215) ;
le capuchon (120) comporte un deuxième canal central (315) se prolongeant à travers un centre du capuchon (120) et une pluralité de trou (220) se prolongeant au moins partiellement à travers le capuchon (120), le deuxième canal central (315) étant configuré pour être accouplé au premier canal central (215), la pluralité de trous (220) étant positionnée de manière circonférentielle autour du deuxième canal central (315) et configurée pour être accouplée à la première pluralité de lumières (225) ; et
la partie de base (125) comporte un troisième canal central (715) se prolongeant à travers un centre de la partie de base (125) et une seconde pluralité de lumières (725) se prolongeant à travers la partie de base (125), le troisième canal central (715) étant configuré pour être accouplé au premier canal central (215), la seconde pluralité de lumières (725) étant positionnée circonférentiellement autour du troisième canal central (715) et configurée pour être accouplée à la première pluralité de lumières (225).

7. Procédé selon l'une quelconque des revendications précédentes, comprenant également le nettoyage de la tige (105), de la partie de base (125) et du capuchon (120) avant la formation de l'applicateur (100).

8. Applicateur de brachythérapie (100) destiné à être inséré dans une cavité corporelle, comprenant :
une tige (105) ayant une première extrémité (110) et une seconde extrémité (115) opposée à la première extrémité, la tige (105) comportant une surface supérieure (200) adjacente à la première extrémité de la tige, la surface supérieure (200) définissant une première rainure (205) et une seconde rainure (210), la seconde rainure (210) circonscrivant la première rainure (205) ; et
un capuchon (120) configuré pour être accouplé à la première extrémité (110) de la tige (105), le capuchon (120) comportant,
une surface d'accouplement (310) configurée pour être accouplée à la surface supérieure (200) de la tige (105),
une première pluralité de saillies (320) se prolongeant à partir de la surface d'accouplement (310), la première pluralité de saillies (320) étant configurée pour être mise en contact avec la première rainure (205), et
une seconde pluralité de saillies (325) se prolongeant à partir de la surface d'accouplement (310) et circonscrivant la première pluralité de saillies (320), la seconde pluralité de saillies (325) étant configurée pour être mise en contact avec la seconde rainure (210).

9. Applicateur de brachythérapie selon la revendication 8, dans lequel :
le capuchon (120) comprend un ou plusieurs éléments de positionnement (330) se prolongeant à partir de la surface d'accouplement (310) ; et
la surface supérieure (200) de la tige (105) définit un ou plusieurs trous (220) configurés pour recevoir les un ou plusieurs éléments de positionnement (330).

10. Applicateur de brachythérapie selon la revendication 8 ou 9, dans lequel :
la tige (105) comporte un premier canal central (215) se prolongeant de la première extrémité (110) à la seconde extrémité (115) et une première pluralité de lumières (225) se prolongeant de la première extrémité (110) à la seconde extrémité (115), la première pluralité de lumières (225) étant positionnée circonférentiellement autour du premier canal central (215) ; et
le capuchon (120) comporte un deuxième canal central (315) se prolongeant à travers un centre du capuchon (120) et une pluralité de trou (335) se prolongeant au moins partiellement à travers le capuchon (120), le deuxième canal central (315) étant configuré pour être accouplé au premier canal central (215), la pluralité de trous (335) étant positionnée de manière circonférentielle autour du deuxième canal central (315) et configurée pour être accouplée à la première pluralité de lumières (225).

11. Applicateur de brachythérapie selon l'une quelconque des revendications 8 à 10, dans lequel la seconde extrémité de la tige comprend une partie de base (125), la partie de base comportant,
une première section (130) configurée pour être accouplée à la seconde extrémité (115) de la tige ;
une deuxième section (135) comportant une surface extérieure (145), la surface extérieure (145) formant un angle (150) avec un axe horizontal (155) à travers la partie de base (125) ;
une troisième section (140), la deuxième section (135) entre la première section (130) et la troisième section (140) ;
un troisième canal central (715) se prolongeant à travers la première section (130), la deuxième section (135) et la troisième section (140) ; et
une seconde pluralité de lumières (725) se prolongeant à travers la première section (130) et la deuxième section (135) de la partie de base (125), et éventuellement l'angle est supérieur ou égal à 45°.

12. Applicateur de brachythérapie selon la revendication 10 ou 11, comprenant également une pluralité de connecteurs (1000) configurés pour être au moins partiellement insérés dans la première pluralité de lumières (725) de la troisième section (140) de la partie de base (125).

13. Applicateur de brachythérapie selon la revendication 12, comprenant également une source de rayonnement (1200) configurée pour être accouplée à la pluralité de connecteurs (1000) et pour délivrer un rayonnement à un emplacement cible.
